# EUROPÄISCHE PATENTSCHRIFT

(11) **EP 0 377 764 B1**
(45) Veröffentlichungstag und Bekanntmachung des Hinweises auf die Patenterteilung: **28.09.1994**
(21) Anmeldenummer: 89100472.3
(22) Anmeldetag: 12.01.1989
(51) Int. Cl.: A61B 5/08, A61B 6/03

(54) **Medizinisches Gerät zur Diagnose und/oder Therapie**
Medical device for diagnostic and/or therapeutic use
Appareil médical destiné au diagnostic et/ou à la thérapie

(43) Veröffentlichungstag der Anmeldung: 18.07.1990
(73) Patentinhaber: SIEMENS AKTIENGESELLSCHAFT, 80333 München (DE)
(72) Erfinder: Kalender, Willi Dr., D-8521 Kleinseebach (DE); Rienmüller, Rainer Prof. Dr. med., D-8000 München 19 (DE); Seissler, Wolfgang, D-8521 Uttenreuth/Weiher (DE)

(56) Entgegenhaltungen:
- DE-A- 3 517 786
- US-A- 3 508 539
- US-A- 3 524 058
- US-A- 3 871 360

## Beschreibung

Die Erfindung betrifft ein medizinisches Gerät zur Diagnose und/oder Therapie mit Mitteln zur Erzeugung von Triggersignalen in Abhängigkeit von der Atemtätigkeit des Patienten.

Abhängig vom Atemzustand verschieben sich Körperorgane und anatomische Strukturen zum Teil erheblich. Bei einer Vielzahl von medizinischen Untersuchungs- und Therapiemethoden ist es daher für befriedigende Ergebnisse erforderlich, die Funktion des Untersuchungs- oder Therapiegerätes mit der Atmung zu synchronisieren, z.B. Einzelmessungen jeweils beim Erreichen eines bestimmten Atemzustandes auszulösen. Diese Forderung besteht z.B. in der Computertomographie für die Anfertigung dynamischer Computertomogramme, bei denen bei jeder Messung einer zeitlichen Folge die gleichen anatomischen Strukturen erfaßt werden sollen. Ebenfalls in der Computertomographie sollen häufig auch Datensätze für dreidimensionale oder multiplanare Rekonstruktionen mit der Atmung synchronisiert werden. Hier werden in zeitlich aufeinanderfolgenden Messungen Schichtbilder in genau definiertem Abstand durch Organe gelegt. Für jede Einzelmessung muß der Atemzustand reproduziert werden, um Organverschiebungen auf Grund der Atmung zwischen den Einzelmessungen auszuschließen. Ähnliche Erfordernisse bestehen z.B. in der Kernspintomographie oder in der Lithotripsie zur Auslösung von Stoßwellen in Abhängigkeit von dem jeweiligen Atemzustand.

Neben der Reproduzierung eines beliebigen Atemzustandes soll häufig auch ein in Absolutwerten genau definierter Atemzustand erreicht werden. Dies ist z.B. bei Lungendichtemessungen von Bedeutung.

Es ist bekannt, den Atemzustand eines Patienten über einen Atemgurt zu erfassen, der über die Brust oder den Bauch gespannt wird und von der Atmung abhängige elektrische Signale erzeugt. Dies stellt keine befriedigende Lösung dar, da keine Absolutmessung der Atemvolumina möglich ist und sich Fehler abhängig davon ergeben, wie stark der Patient Brust- bzw. Zwerchfellatmung einsetzt. Ferner ist es bekannt, die Atemtätigkeit eines Patienten mit Hilfe eines Spirometers oder mit Hilfe von auf den Patienten aufzulegende Elektroden zu erfassen und ein medizinisches Gerät in Abhängigkeit vom erfaßten Wert zu triggern (US-A-3 524 058). Hinsichtlich des Aufbaus des Spirometers ist diesem Dokument nichts entnehmbar. Ferner ist es bekannt, mit Hilfe eines Spirometers eine Lichtquelle in Abhängigkeit von der Atemtätigkeit des Patienten zu bewegen und dadurch Atemsignale auf einem Röntgenfilm aufzuzeichnen (US-A-3 508 539). Für die Triggerung eines medizinischen Gerätes ist diese bekannte Einrichtung nicht geeignet. Schließlich zeigt die DE-A-35 17 786 ein Meßgerät zur Bestimmung des Atemwegwiderstandes mit einem Verschluß für den Atemweg. Für die Messung von Volumina und die Erzeugung von Triggersignalen in Abhängigkeit vom Atemfluß ist dieses bekannte Meßgerät nicht geeignet.

Der Erfindung liegt die Aufgabe zugrunde, ein medizinisches Gerät der eingangs genannten Art so auszubilden, daß eine exakte Erfassung des Atemvolumens bei geringer Fehlerrate möglich ist.

Diese Aufgabe ist erfindungsgemäß gelöst durch die Merkmale des Patentanspruches 1. Bei dem erfindungsgemäßen Gerät atmet der Patient während der gesamten Untersuchungs-oder Therapiedauer über ein Mundstück durch das Spirometer. Dies ist sowohl bei offenen als auch bei geschlossenen Systemen problemlos möglich. Mit dem Spirometer ist eine exakte Erfassung der Vitalkapazität des Patienten möglich. Die Art der Atmung (Brust- bzw. Zwerchfellatmung) ist ohne Auswirkung auf die erzeugten Signale.

Eine zweckmäßige Ausgestaltung der Erfindung besteht darin, daß das Spirometer einen Verschluß für den Atemweg aufweist, der derart gesteuert ist, daß der Atemfluß beim Erreichen eines vorbestimmten Sollwertes kurzzeitig unterbrochen wird. Bei dieser Ausgestaltung kann sichergestellt werden, daß während einer Messung oder eines Therapievorganges praktisch keine Verlagerung von Organen oder anatomischen Strukturen erfolgt.

Die Erfindung ist nachfolgend anhand eines in der Zeichnung dargestellten Ausführungsbeispieles näher erläutert. Es zeigen:
- FIG 1: einen Computertomographen entsprechend der Erfindung und
- FIG 2: ein bei dem Computertomographen der FIG 1 eingesetzes Spirometer.

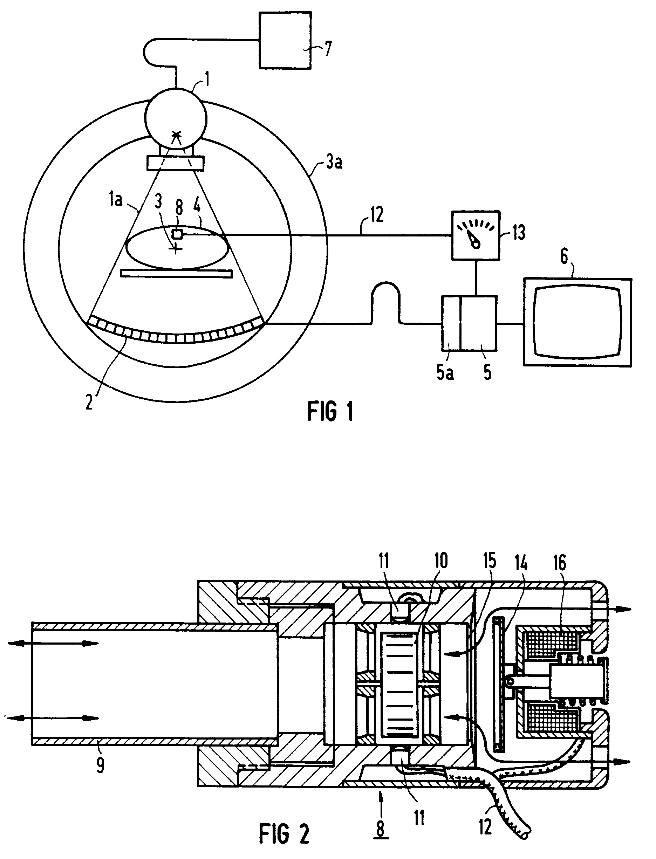

In der FIG 1 ist ein Computertomograph mit einer Röntgenröhre 1 als Strahlenquelle und einem Strahlendetektor 2, der größenordnungsmäßig über 100, z.B. 512 einzelne Detektoren in einer Reihe angeordnet, aufweist, dargestellt. Die Röntgenröhre 1 sendet ein fächerförmiges Strahlenbündel 1a aus, dessen Querschnittsausdehnung senkrecht zur untersuchten Schichtebene gleich der Schichtstärke und in der Schichtebene so groß ist, daß der gesamte Querschnitt des Patienten 4 von Strahlung durchsetzt wird. Der Strahlendetektor 2 ist um den Fokus der Röntgenröhre 1 gekrümmt. Die Meßanordnung 1, 2 ist um eine Achse 3, die etwa mit der Längsachse des Patienten 4 zusammenfällt, mit Hilfe eines Drehrahmens 3a drehbar. Die Anzahl der Detektoren des Strahlendetektors 2 ist der gewünschten Bildauflösung entsprechend gewählt, so daß auf Grund einer Drehung der Meßanordnung 1, 2 von einem Computer 5 die Schwächungswerte einer Bildpunktmatrix der durchstrahlten Transversalschicht des Patienten 4 berechnet und als Bild auf einem Sichtgerät 6 wiedergegeben werden können. Die Röntgenröhre 1 wird von einem Röntgengenerator 7 gespeist.

Jedem Detektorelement des Strahlendetektors 2 ist ein Meßkanal zugeordnet, der zum Computer 5 führt. In diesem Meßkanal sind Verstärkerschaltungen, Multiplexer und Analog/Digital-Wandler vorgesehen, die ein Datenerfassungssystem 5a bilden.

Zur Erfassung der Atemtätigkeit des Patienten 4 und zur Synchronisation der Meßwertbildung mit der Atemtätigkeit atmet der Patient 4 durch ein Spirometer 8. Das Spirometer 8 ist in der FIG 2 genauer dargestellt. Es besitzt ein Mundstück 9 und im Inneren eine Turbine 10, die vom Atemfluß durchströmt wird und in Verbindung mit Meßwertaufnehmern 11 auf der Anschlußleitung 12 vom Atemfluß abhängige elektrische Signale erzeugt. Hierzu kann die Turbine 10 durch Unterbrechung einer Lichtstrecke in einem optoelektronischen System mit den Meßwertaufnehmern 11 elektrische Impulse erzeugen.

Mit dem Spirometer 8 kann die Vitalkapazität des Patienten 4 erfaßt und in einem Rechner 13 (FIG 1) abgespeichert werden. Der Untersucher kann am Rechner 13 eine Triggerschwelle in Prozent der Vitalkapazität oder in Litern oberhalb der Residualkapazität anwählen, bei der die jeweilige Meßwerterfassung erfolgt. Der Rechner 13 vergleicht nach Freigabe der Messung durch den Untersucher das jeweilige Atemvolumen mit dem vorgegebenen Sollwert und sendet beim Erreichen des Sollwertes ein Triggersignal an den Rechner 5, d.h. an das bildgebende System.

Gleichzeitig mit dem Erreichen des Sollwertes soll der Atemfluß mechanisch unterbrochen und damit die Meßwerterzeugung über eine Dauer von bis zu einigen Sekunden bei einem festen Atemzustand durchgeführt werden. Hierzu weist das Spirometer 8 eine über eine Magnetspule 16 in Längsrichtung des Spirometers 8 verstellbare Verschlußplatte 14 auf, die eine Öffnung 15 im Spirometer 8 in ihrer nicht dargestellten, aktiven Stellung verschließt. Nach Abschluß der Meßwerterfassung wird der Atemweg wieder freigegeben. Über entsprechende Sicherheitsabfragen ist sichergestellt, daß der Atemweg nach einigen Sekunden in jedem Fall wieder freigegeben wird.

Der Patient 4 kann, falls dies möglich ist, das Spirometer 8 selbst halten und bei Atemnot die Untersuchung jederzeit aktiv unterbrechen. Die Unterbrechung des Atemflußes birgt also keinerlei Risiko.

## Patentansprüche

1. Medizinisches Gerät zur Diagnose und/oder Therapie mit Mitteln zur Erzeugung von elektrischen Triggersignalen in Abhängigkeit von der Atemtätigkeit des Patienten (4), **dadurch gekennzeichnet,** daß ein von der Atemluft des Patienten (4) durchströmtes Spirometer (8) vorhanden ist, das in einem gemeinsamen Gehäuse eine Turbine (10), die vom Atemfluß durchströmt wird sowie Meßwertaufnehmer (11) für die Erzeugung von vom Atemfluß abhängige elektrische Signale aufweist.

2. Medizinisches Gerät nach Anspruch 1, **dadurch gekennzeichnet,** daß am Geber (10, 11) ein Rechner (13) zur Erzeugung der Triggersignale angeschlossen ist.

3. Medizinisches Gerät nach Anspruch 2, **dadurch gekennzeichnet,** daß am Geber (10, 11) ein Rechner (13) angeschlossen ist, der den jeweiligen Atemzustand an ein bildgebendes System meldet.

4. Medizinisches Gerät nach Anspruch 2 oder 3, **dadurch gekennzeichnet,** daß am Rechner (13) elektrische Stellmittel für die Triggerschwelle angeschlossen sind.

5. Medizinisches Gerät nach einem der Ansprüche 1 bis 4, **dadurch gekennzeichnet,** daß das Spirometer (8) einen Verschluß (14) für den Atemweg aufweist, der derart gesteuert ist, daß der Atemfluß beim Erreichen eines vorbestimmten Sollwertes des Atemvolumens kurzzeitig unterbrochen wird.

## Claims

1. A medical apparatus for diagnosis and/or treatment, comprising means for generating electric trigger signals in dependence on the respiratory activity of the patient (4), characterised in that a spirometer (8) traversed by the air breathed by the patient (4) is present and has a casing containing a turbine (10) traversed by the flow of breath and also containing measured value sensors (11) for generating electric signals depending on the flow of breath.

2. Medical apparatus according to claim 1, characterised in that a computer (13) for generating trigger signals is connected to the transducer (10, 11).

3. Medical apparatus according to claim 2, characterised in that a computer (13) is connected to the transducer (10, 11) and communicates the respective respiratory state to an image-forming system.

4. Medical apparatus according to claim 2 or 3, characterised in that electric means for adjusting the trigger threshold are connected to the computer (13).

5. Medical apparatus according to any of claims 1 to 4, characterised in that the spirometer (8) comprises a means (14) for closing the respiratory tract and controlled so that the flow of breath is briefly interrupted when the volume of breath reaches a predetermined set value.

## Revendications

1. Appareil médical pour le diagnostic et/ou la thérapie, comportant des moyens pour produire des signaux électriques de déclenchement en fonction de l'activité respiratoire du patient (4), caractérisé par le fait qu'il est prévu un spiromètre (8) qui est traversé par l'air respiratoire du patient (4) et qui possède, dans un boîtier commun, une turbine (10) qui est traversée par le flux respiratoire, ainsi que des capteurs de mesure (11) servant à produire des signaux électriques qui sont fonction du flux respiratoire.

2. Appareil médical suivant la revendication 1, caractérisé par le fait qu'un calculateur (13) servant à produire les signaux de déclenchement est raccordé au capteur (10,11).

3. Appareil médical suivant la revendication 2, caractérisé par le fait qu'au capteur (10,11) est raccordé un calculateur (13), qui signale l'état respiratoire respectif à un système d'imagerie.

4. Appareil médical suivant la revendication 2 ou 3, caractérisé par le fait que des moyens de réglage électrique pour le seuil de déclenchement sont raccordés au calculateur (13).

5. Appareil médical suivant l'une des revendications 1 à 4, caractérisé par le fait que le spiromètre (8) possède un système de fermeture (14) pour les voies respiratoires, qui est commandé de telle sorte que le flux respiratoire est interrompu pendant un bref intervalle de temps lorsqu'une valeur de seuil prédéterminée du volume respiratoire est atteinte.
